# EUROPEAN PATENT APPLICATION

(11) **EP 1 518 600 A1**
(43) Date of publication of application: **30.03.2005**
(21) Application number: 04255932.8
(22) Date of filing: 29.09.2004
(51) Int. Cl.: B01J 23/89, C07C 209/36, B01J 37/18, B01J 37/02, B01J 35/00

(54) **Improved nickel catalyst, process for the preparation thereof, process for hydrogenation of m-dinitrobenzene to m-phenylene diamine**

(30) Priority: 29.09.2003 US 671870
(71) Applicant: Council of Scientific and Industrial Research, New Delhi 110 001 (IN)
(72) Inventor: Rode, Chandrashekhar Vasant, Pune 411 008, Maharashtra (IN); Telkar, Manisha Madhukar, Pune 411 008, Maharashtra (IN); Chaudhari, Raghunath Vitthal, Pune 411 008, Maharashtra (IN)
(74) Representative: Tranter, Andrew David

(57) **Abstract**

A nickel based bimetallic catalyst of the formula Ni(x)B(y)/A is disclosed wherein A is a support selected from the group consisting of carbon, alumina, silica, carbonate and zeolite, B is selected from the group consisting of platinum and palladium and x = 0.5 - 99.9%' y=0.2 to 10%, expressed as % of A. A process for the preparation is also disclosed and a process for the hydrogenation using the catalyst to hydrogenate m-dinitro benzene to m-phenylene diamine in the presence of a supported bimetallic platinum catalyst using methanol as a solvent. The catalyst of the present invention is a bimetallic catalyst prepared by precipitation and impregnation technique at very specific preparation conditions.

## Description

### Field of the invention

The present invention relates to a novel nickel catalyst. The present invention also relates to a process for the preparation of an improved nickel catalyst. The present invention also relates to a process for hydrogenation of m-dinitro benzene to m-phenylene diamine using the said improved catalyst. More particularly the present invention relates to a process involving hydrogenation of m-dinitrobenzene to m phenylene diamine in the presence of a supported bimetallic platinum catalyst, using methanol as a solvent. The catalyst system consisting of one metal selected from platinum group and other nickel supported on either carbon or alumina or silica or zeolite. The catalyst of the present invention is a bimetallic catalyst prepared by precipitation and impregnation technique at very specific preparation conditions.

### Background of the invention

Phenylene diamine is an important compound with three isomers- ortho, meta and para-, and are bifunctional compounds used as raw materials for preparation of many chemical intermediates. These isomers are largely used in synthesis of high performance fibers such as polyetherimide, poly (m-phenyleneisophalamide) commonly known as aramid fibers, in preparation of dyes such as Basic Brown, Basic Orange, Direct Black, Developed Black. Phenylene diamine is also used in vulcanization of rubber by sulfur.

In the prior art, there are very few patents on catalyst or its preparation for hydrogenation of dinitro benzene to phenylene diamine. Homogeneous as well as heterogeneous supported catalysts are reported for hydrogenation dinitro benzene. Kvintonics et al. ("Adv. Chem." 173 (1929) 26) reported homogeneous catalyst such Rh (PPh₃)₂Cl in presence of Et₃N for hydrogenation of dinitro benzene. The heterogeneous catalysts mainly consist of palladium catalyst supported on carbon alumina, silica or sepiolite. But majority of these catalysts gives intermediate compound, nitro aniline as the major product rather than phenylene diamine.

Shimazu et al. have described preparation of improved Raney Ni catalyst (JP 9132536) for hydrogenation of dinitro benzene. Raney Ni catalyst is prepared by melting Ni with alumina and cooling quickly to form lumps of catalyst. This improved catalyst on hydrogenation gives a mixture of diamine and nitro aniline.

US patent (US 4185036) describes Pd/C catalyst for hydrogenation of mixture of isomers of dinitro benzene (ortho-, meta- and para-) to give corresponding mixture of nitro aniline. In this process large amount of tar is obtained which not only retards the rate of reaction but also changes the selectivity pattern. Mitsui Toatsu Chemical (JP 59 141 542) reported similar hydrogenation of mixture of isomers of dinitro benzene to their corresponding diamines and nitro anilines by using Pd/C catalyst in methanol as solvent

Palladium or platinum supported on carbon in presence of dimethyl sulfoxide was described by Belous and Rogovik ("Katal. Katal." 15 (1977) 51). The catalyst on hydrogenation gives diamine and m-nitro aniline as the major products. The major drawback is that the life of these catalysts is very low. Pt/C catalyst loses its original activity to half on hydrogenation during its first recycle and the activity drops down to almost one fourth of its original activity in the third recycle.

Berguer et al. in their patent (ES 530565) described Pd catalyst supported on sepiolite for hydrogenation of dinitro benzene in dimethyl formamide as a solvent. The inventor claims 90 times higher rate for Pd sepiolite as compared to Pd/C catalyst. Even this catalyst cannot be recycled as the activity drops down for the third recycle.

Pd on alumina in a mixed solvent system was reported by Bizhanov et al. in their patent SU 1625868. According to the inventors the activity for hydrogenation of dinitro benzene using one solvent system is lower than obtained in mixed solvents system.

Aziz and Vaidyswaran described (IN 158527) continuous hydrogenation of dinitro benzene over fixed bed of catalysts consisting of oxide and sulfide of Ni and W sulfide on alumina. The products obtained were phenylene diamine and nitro aniline.

All the above said catalysts for the hydrogenation of dinitro benzene to phenylene diamine suffer from the disadvantages such as
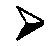 All these catalysts reported do not give complete selectivity to the desired product, phenylene diamine, whereas the intermediate product m-nitro aniline is obtained in higher yield.
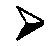 The most of these catalysts lose their activity on recycling, which means that the metal catalysts cannot be recycled, thereby increasing the investment of the process.
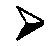 The formation of other isomers as side products have also been reported which demands separation steps for the recovery of the pure products. Hence, the process becomes cost intensive.

The above literature survey shows that the catalyst system reported for hydrogenation of dinitrobenzene consists of a single metal and there is no report available on the use of a bimetallic catalyst. A bimetallic catalyst system is generally known to give several advantages such as longer catalyst life, better productivity and higher selectivity to the desired product. The incorporation of a second suitable metal into a single metal catalyst causes the stabilization of a zero valent state of the first metal, which is essential for the maximum activity, and stability of the hydrogenation catalyst. Also, due to the change in morphology and exposure of active metal sites on the surface, a favorable selectivity pattern can be achieved with the use of a bimetallic catalyst. From this point of view it was thought desirable to develop a bimetallic catalyst system for the hydrogenation of m-dinitro benzene to m-phenylene diamine.

### Objects of the invention

The main object of the present invention is to provide a process for preparation of improved Ni bi-metallic catalyst wherein Ni is in Ni⁰ state.

Another object is to provide a process for the preparation of the said catalyst using milder conditions.

Still another object is to provide an improved process for hydrogenation of m-dinitro benzene to m-phenylene diamine using bimetallic hydrogenation catalyst wherein the Ni would be in Ni⁰ state for selective preparation of m-phenylene diamine comprising of platinum and nickel metal on a suitable support without poisoning at very specific preparation conditions.

Yet another object is to provide the catalyst with higher catalyst life, which can be recycled for number of times without loss of its catalytic activity.

### Summary of the invention

Accordingly, the present invention provides a nickel based bimetallic catalyst of the formula **Ni(x)B(y)/A** wherein A is a support selected from the group consisting of carbon, alumina, silica and zeolite, B is selected from platinum and palladium and x = 0.5-99.9%; y = 0.2 to 10%, expressed as % of A.

The present invention also provides a process for the preparation of a nickel based bimetallic catalyst of the formula **Ni(x)B(y)/A** wherein A is a support selected from the group consisting of carbon, alumina, silica and zeolite, B is selected from platinum and palladium and x = 0.5-99.9%; y = 0.2 to 10% , expressed as % of A, which comprises precipitating a nickel precursor on the support by preparing a slurry of the support in distilled water, heating the slurry to a temperature of at least 60°C and aging at this temperature under constant stirring for at least two hours, adding a solution of a Ni precursor, under constant stirring to this hot slurry, aging the mixture for at least 6 hours, precipitating by adding drop-wise a 10% solution of an alkali carbonate till a pH of at least 9 is attained, cooling the reaction mixture to room temperature and removing the solvent form the resulting slurry to obtain a solid cake, drying the cake to remove the moisture, calcining in an inert or static air atmosphere furnace at 500°C for minimum 10 hours, further reducing the catalyst by molecular hydrogen to obtain the Ni catalyst, impregnating the said Ni catalyst by adding a solution of palladium or platinum, reducing by a reducing agent to obtain bimetallic Ni based catalyst.

In one of embodiment of the invention, the nickel precursor is selected from a nickel salt selected from of acetate, bromide, chloride, and nitrate.

In another embodiment, the source of platinum may be a platinum salt selected from chloride or acetate, preferably a chloride.

In yet another embodiment of the invention, the support source of support may be alumina, silica, zeolite or carbonates of magnesium, calcium and barium, preferably carbon

In still another embodiment of the invention, the precipitation step is carried out in basic medium having pH in the range from 7 to 12, preferably from 8 to 10.

In another embodiment of the invention, the basic medium is obtained using sodium carbonate, potassium carbonate, sodium hydroxide, potassium hydroxide and ammonium carbonate.

In yet another embodiment, the reduction of Ni or Co is carried out in presence of molecular hydrogen at a temperature in the range of 400-800 °C preferably 500-700 °C

In yet another embodiment the reduction of Pt and Pd is carried out by using reducing agent such as hydrazine hydrate or a hydrogen containing gas, preferably formaldehyde.

In a feature of the present invention the hydrogenation of the Nickel catalyst is done in a silica quartz tube placed in a furnace maintained at 500°C at a H₂ flow rate of 5 x 10⁻⁵, m³/min for 10 hours.

The present invention also provides an improved process for hydrogenation of m-dinitro benzene to m-phenylene diamine using the said improved catalyst as mentioned above which comprises hydrogenating the solution of m-dinitro benzene in an organic solvent, under stirring conditions, at temperature ranging between 90-190°C, terminating the reaction, cooling the reaction mixture to room temperature and separating catalyst by conventional methods, isolating the product by distillation.

In one of the embodiments of present invention the organic solvent used may be such as alcohols, dioxanes, ethers exemplified by ethyl or methyl alcohol, 1-4 dioxane, ethyl ether preferably methyl alcohol.

In one of the embodiment of the present invention the concentration of dinitro benzene in reaction mixture using the bimetallic catalyst is in the range of 10-70%, more preferably between 15-50%.

In another embodiment the hydrogenation process may be carried out under hydrogen pressure of 5-100 bar, more preferably between 10 - 80 bar.

In another embodiment the reaction temperatures may be in the range of 20°-200°C, more preferably between 80° -190°C.

In a feature of the present invention the conversion achieved are almost 100% for dinitro benzene with 98-100% selectivity for phenylene diamines at milder process conditions.

In still another feature said catalyst can be reused for 5 runs without losing its activity.

### Detailed description of the invention

It was observed during the investigation into this invention, that the Ni catalysts used in the prior art processes are in Ni⁺² state and because of this state it is observed that the selectivity for the m-dinitrobenzene is low. It is further observed that by using milder conditions as provided in the present invention, the Ni in the catalyst would be in Ni⁰ state, which gives improved activity and thereby enhanced selectivity for the hydrogenation reactions.

The monometallic Ni and bimetallic Ni-Pt supported on carbon were tested for their activity and selectivity to m-phenylene diamine. The activity obtained for bimetallic catalyst (10%Ni-0.25 %Pt/C) was ten times higher than the monometallic Ni (10% Ni/C) catalyst. These catalysts were characterized by XRD and XPS. Monometallic Ni catalyst (10% Ni/C) showed presence of Ni⁰ and Ni⁺² species, where as 10%Ni-0.25 %Pt/C showed presence of only Ni⁰ species.

The bimetallic catalyst prepared of the present invention gives 100% conversion of m-dinitro benzene with >99 % selectivity to m-phenylene diamine at milder conditions. The catalyst can be reused for more than 5 runs without losing its activity. The desired product, m-phenylene diamine in its pure form can be recovered, by separating the catalyst merely by filtration.

Neither of monometallic Ni or Pt catalysts gives higher activity, selectivity nor stability as compared to said Ni-Pt bimetallic catalyst

The present invention provides a process for the preparation of an improved nickel bimetallic which comprises catalyst comprises precipitating a nickel precursor on a support by preparing the slurry of the support in distilled water, heating the slurry to a minimum temperature of 60°C and aging at this temperature under constant stirring for at least two hours, adding to this hot slurry, a solution of a Ni precursor, under constant stirring aging the mixture of at least 6 hours, precipitating by adding drop-wise a 10% solution of an alkali carbonate till a minimum pH of 9 is attained, cooling the reaction mixture to room temperature and removing the solvent form the resulting slurry to obtain a solid cake, drying the cake to remove the moisture, calcining in an inert or static air atmosphere furnace at 500°C for minimum 10 hours, further reducing the catalyst by molecular hydrogen to obtain the Ni catalyst, impregnating the said Ni catalyst by adding a solution of H₂PtCl₄, reducing by a reducing agent to obtain bimetallic Ni-Pt catalyst.

The Ni bimetallic catalyst has the general formula **Ni(x)B(y)/A** where A is a support, such as carbon, alumina, silica, zeolite, B is platinum or palladium and x = 0.5-99.9%; y = 0.2 to 10%, expressed as % of A and characterized by XRD as given in table 1

**Table 1:**

| **XRD results of mono and bimetallic Ni catalysts** | | | |
|---|---|---|---|
| Catalyst | Element for detection | 2θ (degree) | |
| | | Observed value | Literature value* ²⁰ |
| 10% Ni/C | Ni | 37.9 | 37.6 |
| | | 44.5 | 44.5 |
| | | 51.5 | 51.8 |
| | | 73.3 | 73.6 |
| | C | 26.06 | 26.18 |
| | | 43.7 | 43.72 |
| 0.25% Pt/C | Pt | 39.0 | 39.40 |
| | | 45.0 | 45.49 |
| | | 62.0 | 63.96 |
| 10%Ni-0.25% Pt/C | Ni | 44.5 | 44.5 |
| | | 51.5 | 51.8 |
| | | 73.3 | 73.6 |
| | | 39.0 | 39.40 |
| | Pt | 45.0 | 45.49 |
| | | 62.0 | 63.96 |
| and XPS as given in table-2 | | | |

**Table 2:**

| **XPS results of mono and bimetallic Ni catalysts** | | | |
|---|---|---|---|
| Catalyst | Element | Binding Energy, eV | |
| | | Observed values | Literature values²¹ |
| 10%Ni/C | Ni | 852.3 | 852 (Shows presence of Ni⁰) |
| | | 853.3 | 853 (Shows presence of Ni²⁺) |
| 10%Ni-0.25% Pt/C | Ni | 852 | 852 |

The source of nickel may be salt of acetate, bromide, chlorides preferably a nitrate. The source of platinum may be salt or chloride of acetate of platinum preferably a chloride. The source of support may be alumina, silica, zeolite or carbonates of magnesium, calcium and barium, preferably carbon. The catalyst preparation precipitation step is carried out in basic medium having pH in the range from 7 to 12, preferably from 8 to 10.

The alkali carbonate can be sodium carbonate, potassium carbonate, sodium hydroxide potassium hydroxide, and preferably ammonium carbonate. The reduction of Ni or Co is carried out in presence of molecular hydrogen in the temperature range of 400-800 °C preferably 500-700 °C

The reduction of Pt and Pd is carried out by using reducing agent such as hydrazine hydrate, hydrogen containing gas, preferably formaldehyde. In a feature of the present invention the hydrogenation of the Nickel catalyst is done in a silica quartz tube placed in a furnace maintained at 500°C at a H₂ flow rate of 5 x 10⁻⁵, m³/min for 10 hours.

The present invention also provides an improved process for hydrogenation of m-dinitro benzene to m-phenylene diamine using the said improved catalyst as mentioned above which comprises hydrogenating the solution of m-dinitro benzene in an organic solvent, under stirring conditions, at temperature ranging between 90-190°C, terminating the reaction, cooling the reaction mixture to room temperature and separating catalyst by conventional methods, isolating the product by distillation.

The organic solvent used can be alcohols, dioxanes, ethers exemplified by ethyl or methyl alcohol, 1-4 dioxane, ethyl ether preferably methyl alcohol. The concentration of dinitro benzene in the reaction mixture using the bimetallic catalyst is in the range of 10-70%, more preferably between 15-50%. The hydrogenation process can be carried out under hydrogen pressure of 5-100 bar, more preferably between 10 - 80 bar. The reaction temperatures may be in the range of 20°-200°C, more preferably between 80° -190°C.

In a feature of the present invention the conversion achieved are almost 100% for dinitro benzene with 98-100% selectivity for phenylene diamines at milder process conditions. In another feature said catalyst can be reused for 5 runs without losing its activity.

The process of the invention is described hereinbelow in details with examples, which are illustrative only and should not be considered to limit the scope of the invention in any manner.

### Example 1

This example illustrates the preparation of 10%Ni-1% Pt / C catalyst by the following procedure.

Support is calcined in a static furnace at 773 K for 4 hours. Slurry of the support (10 gms) was made in distilled water and stirred for 2 hours at 363 K. To this hot slurry, a solution of Ni (NO₃)₂.6H₂O (4.95 gms in10 ml of water) was added. After stirring for 6 hours, 10 % of ammonium carbonate solution was added dropwise till pH value of 9 was attained. The resulting slurry was filtered to obtain a solid cake. The AAS analysis revealed absence of Ni in the filtrate indicating complete precipitation of Ni as Ni carbonate. The cake was dried overnight at 383 K and calcined in a static air furnace at 773 K for 10 hours. The reduction of the catalyst was carried out in an activation furnace in a silica quartz tube at 773 K at H₂ flow rate of 5 x 10⁻⁵, m³/min for 10 hours.

The above Ni catalyst was added to a solution of chloroplatinic acid (prepared by dissolving of 0.172 gms PtCl₄ in 6ml of dilute HCl), precursor of Pt. This suspension is refluxed for 4 hours and then 6ml of formaldehyde is added as reducing agent in two steps. This solution is further stirred for 2 hours and then filtered to give a bimetallic 10 %Ni-1%Pt/C catalyst. The catalyst obtained by above process was characterized by XRD and XPS

### Example 2

This example illustrates the preparation of 10%Ni-0.5% Pt / C catalyst by the following procedure.

Support is calcined in a static furnace at 773 K for 4 hours. Slurry of the support (10 gms) was made in distilled water and stirred for 2 hours at 363 K. To this hot slurry, a solution of Ni (NO₃)₂.6H₂O (4.95 gms in10 ml of water) was added. After stirring for 6 hours, 10 % of ammonium carbonate solution was added dropwise till pH value of 9 was attained. The resulting slurry was filtered to obtain a solid cake. The AAS analysis revealed absence of Ni in the filtrate indicating complete precipitation of Ni as Ni carbonate. The cake was dried overnight at 383 K and calcined in a static air furnace at 773 K for 10 hours. The reduction of the catalyst was carried out in an activation furnace in a silica quartz tube at 773 K at H₂ flow rate of 5 x 10⁻⁵, m³/min for 10 hours.

The above Ni catalyst was added to a solution of chloroplatinic acid (prepared by dissolving of 0.085 gms PtCl₄ in 6ml of dilute HCl), precursor of Pt. This suspension is refluxed for 4 hours and then 6ml of formaldehyde is added as reducing agent in two steps. This solution is further stirred for 2 hours and then filtered to give a bimetallic 10 %Ni-0.5%Pt/C catalyst. The catalyst obtained by above process was characterized by XRD and XPS

### Example 3

This example illustrates the performance in terms of TOF hr⁻¹, (Turn over frequency) and selectivity to m-phenylene diamine of the bimetallic Ni-Pt/C catalyst as described in Examples 1-4 for the hydrogenation of dinitro benzene to phenylene diamine as follows.

| Example No. | Catalyst | TOF, (hr⁻¹) | Sel.to m phenylene diamine (%) |
|---|---|---|---|
| 1 | 10 %Ni-1%Pt/C | 200 | 99.2 |
| 2 | 5 %Ni-1%Pt/C | 165 | 99.25 |
| 3 | 10 %Ni-0.5%Pt/C | 150 | 99.5 |
| 4 | 10%Ni-0.25%Pt/C | 111.4 | 99.2 |

**Reaction conditions:** Temperature:120°C; Pressure: 34 bar; Solvent: Methanol; Concentration of DNB: 7.5 gms; Concentration of Catalyst: 0.13 gms; Agitation Speed: 13.3 Hz; Liquid Volume: 150 ml.

### Example 4

This example illustrates the performance of 10 %Ni -0. 25%Pt/ C in hydrogenation of m-dinitro benzene to m-phenylenediamine at different temperatures. The reaction in presence of this catalyst was carried out as per the procedure given earlier.

| Temp, °C | TOP, hr⁻¹ | Selectivity to m phenylene diamine, % |
|---|---|---|
| 70 | 9 | 73 |
| 100 | 35 | 89 |
| 120 | 111.4 | 99 |
| 150 | 149 | 99 |

**Reaction conditions:** Pressure: 34 bar; Solvent: Methanol; Concentration of DNB: 7.5 gms; Concentration of Catalyst: 0.13 gms; Agitation Speed: 13.3 Hz; Liquid Volume: 150 ml.

### Example 5

This example illustrates the performance of 10%Ni-0.25%Pt/C catalyst for hydrogenation of m-dinitro benzene to m-phenylenediamine at different pressure conditions. The reaction in presence of this catalyst was carried out as per the procedure given earlier.

| Pressure, bar | TOF , hr⁻¹ | Selectivity to m phenylene diamine, % |
|---|---|---|
| 2.067 | 25 | 86 |
| 3.44 | 111.4 | 99 |
| 4.82 | 145 | 99.6 |
| 6.89 | 159 | 99.9 |

**Reaction conditions:** Temperature: 120⁰C; Pressure: 34 bar; Solvent: Methanol; Concentration of DNB: 7.5 gms; Concentration of Catalyst: 0.13 gms; Agitation Speed: 13.3 Hz; Liquid Volume: 150 ml

### Example 6

This example illustrates the reusability bimetallic 10%Ni-0.25%Pt/C catalyst over mono Ni and Pt catalyst for hydrogenation of m-dinitro benzene to m-phenylenediamine. The reaction in presence of this catalyst was carried out as per the procedure given earlier.

| Sr. No. | Catalyst | TOF, hr⁻¹ | Selectivity, % | |
|---|---|---|---|---|
| | | | MPD | mNA |
| 1. | 10 % Ni/ C (Fresh) | 11.23 | 46 | 54 |
| | Recycle 1 | 9.0 | 40 | 60 |
| | Recycle 2 | 4.8 | 35 | 65 |
| | Recycle 3 | 2.8 | 10 | 90 |
| 2. | 10% Ni-0.25 %Pt/C (Fresh) | 111.4 | 99.2 | 0.0 |
| | Recycle 1 | 111.0 | 99.6 | 0.0 |
| | Recycle 2 | 111.0 | 99.1 | 0.0 |
| | Recycle 3 | 110.5 | 99.2 | 0.0 |
| | Recycle 4 | 110.5 | 99.2 | 0.0 |
| | Recycle 5 | 110 | 99.2 | 0.0 |
| 3. | 10% Ni -0.5 %Pt/C (Fresh) | 150 | 99.5 | 0.0 |
| | Recycle 1 | 149 | 99.2 | 0.0 |
| | Recycle 2 | 148 | 99.1 | 0.0 |
| | Recycle 3 | 148 | 99.1 | 0.0 |
| | Recycle 4 | 148 | 99.2 | 0.0 |
| | Recycle 5 | 148 | 99.2 | 0.0 |
| 4. | 10% Ni -1 %Pt/C (Fresh) | 200 | 99.2 | 0.0 |
| | Recycle 1 | 198.5 | 99.3 | 0.0 |
| | Recycle 2 | 198 | 99.3 | 0.0 |
| | Recycle 3 | 198 | 99.0 | 0.0 |
| | Recycle 4 | 198 | 99.0 | 0.0 |
| | Recycle 5 | 198 | 99.0 | 0.0 |
| 5. | 0.25 % Pt/C (Fresh) | 121 | 75.0 | 25 |
| | Recycle 1 | 100 | 63 | 37 |
| | Recycle 2 | 70 | 35 | 65 |
| | Recycle 3 | 36 | 0.0 | 99.2 |
| 6. | 1 % Pt/C (Fresh) | 125 | 78 | 22 |
| | Recycle 1 | 98 | 60 | 40 |
| | Recycle 2 | 65 | 23 | 77 |
| | Recycle 3 | 30 | 0.0 | 99.0 |

### The present invention gives following advantages over any other known processes

- Selective hydrogenation of dinitro benzene to phenylene diamine is achieved using novel bimetallic catalyst described above which cannot be obtained by corresponding monometallic catalyst
- Bimetallic Ni-Pt catalyst described in said process gives 10 times higher activity (TOF) as compared to monometallic Ni catalyst
- The activity of bimetallic Ni-Pt catalyst described in said process remains constant even after 5 recycles where as for monometallic Ni catalyst the activity obtained for second recycle is one half of its original activity.
- Results obtained under milder reaction conditions.
- Easy separation of the product m-phenylene diamine in the pure from. The catalyst can be easily separated from the reaction mixture.

## Claims

1. A nickel based bimetallic catalyst of the formula **Ni(x)B(y)/A** wherein A is a support selected from the group consisting of carbon, alumina, silica, carbonate and zeolite, B is selected from the group consisting of platinum and palladium and x = 0.5-99.9%; y = 0.2 to 10% , expressed as % of A.

2. A process for the preparation of a nickel based bimetallic catalyst of the formula **Ni(x)B(y)/A** wherein A is a support selected from the group consisting of carbon, alumina, silica, carbonates and zeolite, B is selected from platinum and palladium and x = 0.5-99.9%; y = 0.2 to 10%, expressed as % of A, which comprises precipitating a nickel precursor on the support by preparing a slurry of the support in distilled water, heating the slurry to a temperature of at least 60°C and aging at this temperature under constant stirring for at least two hours, adding a solution of a Ni precursor, under constant stirring to this hot slurry, aging the mixture, bringing the pH of the mixture to a range of 7 to 12 in order to obtain a precipitate, cooling the reaction mixture to room temperature and removing the solvent form the resulting slurry to obtain a solid cake, drying the cake to remove the moisture, calcining in an inert or static air atmosphere, further reducing the catalyst by molecular hydrogen to obtain the Ni catalyst, impregnating the said Ni catalyst by adding a palladium or platinum source, reducing by a reducing agent to obtain bimetallic Ni based catalyst.

3. A process as claimed in claim 2 wherein the nickel precursor and support slurry mixture is aged for at least 6 hours.

4. A process as claimed in claim 2 wherein the nickel precursor is a nickel salt selected from the group consisting of acetate, bromide, chloride and nitrate.

5. A process as claimed in claim 2 wherein the source of platinum is a platinum salt selected from the group consisting of chloride and acetate.

6. A process as claimed in claim 2 wherein the source of platinum is H₂PtCl₄

7. A process as claimed in claim 2 wherein the support is selected from the group consisting of alumina, silica, zeolite, carbonates of magnesium, calcium and barium and carbon.

8. A process as claimed in claim 2 wherein the precipitation step is carried out in basic medium having pH in the range from 7 to 12.

9. A process as claimed in claim 8 wherein the pH during precipitation is 9.

10. A process as claimed in claim 2 wherein the precipitating by adding drop-wise a solution of sodium carbonate, potassium carbonate, sodium hydroxide, potassium hydroxide and ammonium carbonate.

11. A process as claimed in claim 2 wherein the alkali carbonate is added in a 10% solution.

12. A process as claimed in claim 2 wherein the calcination is carried out in a furnace at 500°C for at least 10 hours.

13. A process as claimed in claim 2 wherein the reduction of Ni or Co is carried out in presence of molecular hydrogen at a temperature in the range of 400-800°C, preferably 500-700 °C

14. A process as claimed in claim 2 wherein the reduction of Pt and Pd is carried out by using reducing agent selected from the group consisting of hydrazine hydrate, a hydrogen containing gas and formaldehyde.

15. A process as claimed in claim 2 wherein the hydrogenation of the Nickel catalyst is done in a silica quartz tube placed in a furnace maintained at 500°C at a H₂ flow rate of 5 x 10⁻⁵, m³/min for 10 hours.

16. A process for hydrogenation of m-dinitro benzene to m-phenylene diamine using the said improved catalyst as mentioned above which comprises hydrogenating the solution of m-dinitro benzene in an organic solvent, under stirring conditions, at temperature ranging between 90-190°C, terminating the reaction, cooling the reaction mixture to room temperature and separating catalyst by conventional methods, isolating the product by distillation.

17. A process as claimed in claim 16 wherein the organic solvent used is selected from an alcohol, dioxane and an ether.

18. A process as claimed in claim 17 wherein the organic solvent used is selected from ethyl alcohol, methyl alcohol, 1-4 dioxane and ethyl ether.

19. A process as claimed in claim 16 wherein the concentration of dinitro benzene in reaction mixture using the bimetallic catalyst is in the range of 10-70%, more preferably between 15-50%.

20. A process as claimed in claim 16 wherein the hydrogenation process is carried out under hydrogen pressure of 5-100 bar, more preferably between 10 - 80 bar.

21. A process as claimed in claim 16 wherein the reaction temperatures is in the range of 20°-200°C, more preferably between 80° -190°C.

22. A process as claimed in claim 16 wherein the conversion achieved is almost 100% for dinitro benzene with 98-100% selectivity for phenylene diamines at milder process conditions:

23. A process as claimed in claim 16 wherein the catalyst is reused for 5 runs without losing its activity.
